# EUROPEAN PATENT SPECIFICATION

(11) **EP 1 575 533 B1**
(45) Date of publication and mention of the grant of the patent: **03.06.2009**
(21) Application number: 03814846.6
(22) Date of filing: 16.12.2003
(51) Int. Cl.: A61K 31/185, A61Q 19/00, A61K 8/368, A61K 8/97, A61K 36/185, A61K 36/48, A61K 31/07, A61K 31/203, A61K 36/236, A61K 45/06, A61P 17/00

(54) **TOPICAL COMPOSITIONS HAVING A NATURAL INGREDIENT AND METHOD OF USE**
TOPISCHE ZUSAMMENSETZUNGEN MIT EINEM NATÜRLICHEN INHALTSSTOFF UND ANWENDUNGSVERFAHREN
COMPOSITIONS TOPIQUES COMPRENANT UN CONSTITUANT NATUREL ET LEUR PROCEDE D'UTILISATION

(30) Priority: 26.12.2002 US 330040
(43) Date of publication of application: 21.09.2005
(73) Proprietor: AVON PRODUCTS, INC., New York, NY 10020-1196 (US)
(72) Inventor: HINES, Michelle, D., Hickory Creek, TX 75065-3611 (US); LU, Michelle Z., Shanghai, 200050 (CN); AGINSKY, Jill, Union, New Jersey 07083-3842 (US); DRYER, Laurence, Butler, New Jersey 07405 (US); PTCHELINTSEV, Dmitri, Jersey City, NJ 07302 (US)
(74) Representative: Dr. Weitzel & Partner
(86) International application number: PCT/US2003/040122
(87) International publication number: WO 2004/060288

(56) References cited:
- EP-A1- 0 933 077
- EP-A2- 1 000 613
- WO-A2-20/04060285
- JP-A- 5 124 952
- JP-A- 6 080 553
- US-A- 5 958 395
- US-A1- 2004 126 344
- US-B1- 6 436 417

## Description

### RELATED APPLICATIONS

This is a continuation-in-part application that claims priority of co-pending United States Patent Application Serial No. 10/330,040, filed December 26, 2002.

### BACKGROUND OF THE INVENTION

### 1. Field of the Invention

The present invention relates to topical compositions having an active ingredient derived from a plant or plant material. More particularly, the present invention relates to topical compositions that improve the appearance of skin, especially by alleviating skin irritation, with the topical compositions having at least one natural plant extract that inhibits COX-2 enzyme, NGF protein and/or TNF-alpha protein activity. Still more particularly, the present invention relates to using the topical compositions of the present invention.

### 2. Description of the Related Art

Active ingredients derived from plants have over time been employed in topical compositions for a wide variety of medicinal, therapeutic and cosmetic purposes. Such actives can be obtained from various parts of a plant such as seeds, leaves, roots, bark, flowers, cones, stems, rhizomes, callus cells, protoplasts, organs and organ systems, and meristems. Active ingredients are incorporated in such compositions in a variety of forms. Such forms include a pure or semi-pure component, a solid or liquid extract or derivative, or solid plant matter. Plant matter may be minced, ground, crushed or otherwise physically modified for incorporation into a composition.

A problem commonly encountered when using an active ingredient derived from a plant or plant part is the relatively low level at which they are naturally present. Such low levels frequently require relatively large amounts of plant leaf/tissue or seed be processed in order to obtain desired or useful quantities of active ingredients. For rare plants or plant parts, such large amounts may be unavailable or difficult to obtain.

Currently, a wide variety of topically applied pharmaceutical and cosmetic products are in commercial use. For example, there is active contemporary interest in the cosmetics industry to develop products that may be applied topically to the skin that provide anti-aging, hydrating, and/or skin texturing benefits. Cosmetic products that enhance the appearance of skin are increasingly in demand. Consumers are interested in mitigating or delaying the signs of chronologically, hormonally and/or photo-aged skin, such as fine lines, wrinkles, dry skin, and sagging skin. During the aging process, the complexion of the skin, i.e., the color and appearance of the skin, deteriorates slowly from intrinsic aging and/or exposure to sunlight. Cosmetic surgery can be used as a treatment for aged skin, but such treatment is costly and carries the risks normally associated with anesthesia and surgery. Alternatively, cosmetic products that are able to provide anti-aging or other skin-care benefits are highly desired by consumers. However, one problem that arises with pharmaceutical and cosmetic topically applied products is that the active ingredient or ingredients are often irritating to the skin. This side effect may limit the use of, or the concentration of, certain cosmetic or pharmaceutical active ingredients.

The number of cosmetic skin care products is steadily increasing. Commonly, such products contain organic acids or other materials as active ingredients. Such active ingredients include, for example, hydroxylated acids and their derivatives, such as omega-hydroxy acids (i.e., undecanoic acid), α-hydroxy acids (i.e., lactic, glycolic, citric), β-hydroxy acids (i.e., salicylic, 5-n-octanoylsalicylic), and retinoids (i.e., retinoic acids, retinol). It is known that these active ingredients have a significant disadvantage in that they frequently are associated with consumer skin irritation or discomfort characterized by burning, smarting, itching or sensation of tightness after application. There remains a general need in both the cosmetics industry and pharmaceutical industry for topically applied products containing various active ingredients that are effective without producing the undesirable side effect of skin irritation. It is known that a significant number of consumers have sensitive skin or are susceptible to allergic skin reactions when topically applied products are used. For example, products having certain surfactants, preservatives, fragrances and the like, as well as active ingredients, have skin-irritant characteristics.

More particularly, in view of the previous discussion of demands and limitations in the cosmetics industry, there remains a need for topically applied, cosmetic compositions that have skin benefits without skin irritation as a side effect using natural ingredients as active components.

Treatments of irritable skin or skin inflammation are known.

US 5 993 833 discloses administering a composition having an antagonist compound. US 6 143 303 discloses an anti-inflammatory, analgesic composition comprising an extract of the plants Dodonaea petiolaris and dodonaea viscose. EP-A-1 243 250 relates to fibres as anti-irritant agents in a cosmectic or dermatologic composition.

In spite of the various pharmaceutical and cosmetic products on the market that are topically applied to skin, there remains a need for effective topically applied compositions that incorporate natural plant extracts or synthesized forms of natural plant extracts to provide an improved aesthetic appearance to the skin, especially inflamed skin, or to achieve the benefits of active ingredients contained in the composition with mitigation or elimination of irritant side effects occasioned by the use such actives.

### SUMMARY OF THE INVENTION

It is an object of the present invention to provide cosmetic compositions that improve the appearance of skin, including remediating the effects of aging.

It is another object of the present invention to provide topical cosmetic or pharmaceutical compositions having a plant-derived skin anti-irritant active ingredient or blends of plant-derived skin anti-irritant active ingredients in a pharmaceutically or cosmetically acceptable vehicle.

It is still another object of the present invention to provide a topical composition that delivers a skin anti-irritant active ingredient derived from a plant extract together with an effective level of a cosmetic, dermatologic, or pharmaceutical active ingredient.

It is yet another object of the present invention to provide topical compositions having a plant extract inhibitory to COX-2 enzyme, NGF protein and/or TNF-alpha protein.

It is a further object of the present invention to provide methods for topically applying such compositions.

It is a still further object of the present invention to provide methods of improving the appearance of skin, including remediating the effects of aging, and treating skin irritation symptoms or conditions by topically applying the compositions of the invention to the skin are also provided.

These and other objects and advantages of the present invention, and equivalents thereof, are achieved by anti-irritant cosmetic and pharmaceutical compositions having a botanical extract or blends of botanical extracts, and use of such compositions for topical application.

Topical compositions for treating skin to improve the aesthetic appearance of the skin and/or to provide anti-aging benefits to the skin are provided. Also provided are topical compositions for treating skin, the treatment producing attenuated irritation, preferably no visible irritation, to the skin.

Topical compositions for treating the symptoms of skin irritation or for treating skin conditions that elicit an irritation response from the skin are also provided. These compositions have a cosmetically, dermatologically, or pharmaceutically effective amount of at least one plant extract sufficient to inhibit COX-2 enzyme, NGF protein and/or TNF-alpha protein, and a cosmetically, dermatologically, or pharmaceutically acceptable vehicle.

The compositions of the present invention have at least one plant extract. The at least one plant extract is any of the following: *Ilex purpurea* Hassk, *Ligusticum chiangxiong, Osmunda japonica, Ligusticum lucidum, Butea frondosa*, or *Mimusops elengi*. Preferably, the at least one plant extract is any of the following: *Ilex purpurea* Hassk, *Ligusticum chiangxiong, Osmunda japonica, Butea frondosa*, or *Ligusticum lucidum.*

The present compositions alleviate irritation symptoms or conditions, including, but not limited to: erythema, psoriasis, edema, acne, warts, hyper-pigmentation, hypo-pigmentation, wheeling, blotchiness, uneven skin tone, scaling, flaking, itching, burning, stinging, tingling, numbing, wind irritation, temperature irritation, smoke irritation, and chemical irritation.

### DETAILED DESCRIPTION OF THE INVENTION

The present invention provides topical compositions having a plant extract or blends of plant extracts, preferably natural plant extracts, that alleviate irritation of the skin, including lips, particularly irritation caused by active ingredients in cosmetic, dermatologic or pharmaceutical products. The compositions of the present invention provide benefits to a variety of skin irritation symptoms or conditions.

The present invention also provides for compositions to improve the aesthetic appearance of skin, including remediating the effects of aging. These benefits are manifest by one or more of the following: reduction in pore size; improvement in skin tone, radiance, clarity and/or tautness; promotion of anti-oxidant activity; improvement in skin firmness, plumpness, suppleness, and/or softness; improvement in procollagen and/or collagen production; improvement in skin texture and/or promotion of retexturization; improvement in skin barrier repair and/or function; improvement in appearance of skin contours; restoration of skin luster and/or brightness; replenishment of essential nutrients and/or constituents in the skin decreased by aging and/or menopause; improvement in communication among skin cells; increase in cell proliferation and/or multiplication; increase in skin cell metabolism decreased by aging and/or menopause; improvement in skin moisturization; promotion and/or acceleration of cell turnover; enhancement of skin thickness; reducing skin sensitivity; increase in skin elasticity and/or resiliency; and enhancement of exfoliation, with or without the use of alpha or beta hydroxy acids, keto acids or other exfoliants. Accordingly, the method of the present invention provides an effective amount from 0.0001 wt% to 20 wt% by weight of the composition, and an effective in the treatment of one or more of the following: reducing or preventing loss of collagen; improving skin firmness/plumpness; improving skin texture; decreasing/preventing lines and wrinkles; improving skin tone; enhancing skin thickness; decreasing pore size; reducing skin discoloration; reducing acne; and reducing skin sensitivity.

The present invention also provides for compositions that provide an anti-aging benefit. The compositions have an effective amount of one or more ingredients which, when applied to human skin, prevent, treat and/or ameliorate the various signs of aging at the area or portion of skin to which they are applied. In particular, the present invention provides compositions and methods for treating skin to prevent, inhibit, reduce and/or ameliorate the signs of dermatological aging due to, for example, chronological aging, hormonal aging, and/or photoaging. Such signs of aging include, but are not limited to skin fragility; loss of collagen and/or elastin; estrogen imbalance in skin; skin atrophy; appearance and/or depth of lines and/or wrinkles, including fine lines; skin discoloration, including dark eye circles; skin sagging; skin fatigue and/or stress, e.g., skin breakout due to environmental stress, such as pollution and/or temperature changes; skin dryness; skin flakiness; cellular aging; loss of skin tone, elasticity and/or luster; loss of skin firmness; poor skin texture; loss of skin elasticity and/or resiliency; and thin skin.

To improve the aesthetic appearance of skin, these compositions have at least one of the natural plant extracts or synthesized forms of the natural plant extracts of the present invention; namely, *Ilex purpurea Hassk, Ligusticum chiangxiong, Osmunda japonica, Ligusticum lucidum, Butea frondosa,* or *Mimusops elengi.* Preferably, the at least one plant extract is any of the following: *Ilex purpurea Hassk, Ligusticum chiangxiong, Osmunda japonica*, or *Ligusticum lucidum*. Also preferably, these compositions also have one or more cosmetically active agents as known in the cosmetic field, including but not limited to the cosmetic agents enumerated herein, that provide one or more of the above-identified skin appearance benefits.

Skin irritation may result from a variety of physical or chemical factors, including environmental factors such as exposure to wind, heat or cold, air pollutants, and cigarette smoke. Cosmetic and pharmaceutical products may have ingredients or combinations of ingredients that produce visible skin irritation as a side effect. Susceptibility to skin irritation may vary from individual to individual, and frequently limits the use of certain products or the use of concentrations of active ingredients that might produce more advantageous results at higher levels but for the production of skin irritation as a side-effect. Skin irritation symptoms or conditions include, but are not limited to, erythema, psoriasis, edema, hyper-pigmentation, hypo-pigmentation, acne, warts, wheeling, blotchiness, uneven skin tone, scaling, flaking, itching or pruritus, tightness, burning, prickling, stinging, tingling, numbing, wind irritation, temperature irritation, smoke irritation, chemical irritation, or any combinations thereof.

Cosmetic, dermatological and pharmaceutical products commonly have an active agent or agents that produce skin irritation. Examples of active agents having skin irritation as a side effect include, but are not limited to, hydroxylated acids and α-hydroxy acids (i.e., lactic, glycolic, citric, malic, tartaric, mandelic, gluconic, methyl lactic, phenyl lactic, atrolactic, glyceric, benzilic, z-hydroxyheptanoic, z-hydroxyoctanoic and any combinations thereof), β-hydroxy acids (i.e., salicylic, 5-n-octanoylsalicylic and other derivatives of salicylic), retinoids (retinoic acid; retinol and its esters); anthralins (i.e., dioxyanthranol), anthranoids, peroxides (i.e., benzoyl peroxide), minoxidil, lithium salts, antimetabolites, vitamin D, hair dyes or colorants (i.e., para-phenylenediamine; aminophenols), alcoholic perfuming solutions (i.e., perfumes; toilet waters; aftershaves; deodorants), antiperspirant agents (i.e., some aluminum salts), depilatory or hair permanent active agents (i.e., thiols), depigmentating agents (i.e., hydroquinone), and some insecticide active agents. If topical products had anti-irritant protection, it would be possible to increase the amount of the normal irritant active agent (i.e., AHA or BHA) in the product without producing unpleasant skin irritation or irritation side effects. The use of the present compositions makes it possible to improve the efficacy of cosmetic, dermatological or pharmaceutical products by increasing the concentration or amount of cosmetic, dermatological, or pharmaceutical active agent as compared to the amount or concentration of such agent normally used.

It has now been found that the addition of plant extracts antagonistic to tumor necrosis factor alpha (TNF-alpha) protein, nerve growth factor (NGF) protein and/or cyclooxygenase-2 (COX-2) enzyme to topical cosmetic, dermatological, or pharmaceutical compositions, preferably compositions having skin-irritant ingredient(s), alleviates or even eliminates skin irritation.

It is known that there are two different isoforms of cyclooxygenase (COX) enzymes, namely COX-1 and COX-2. COX-1 is present in nearly all parts of the body at a constant level and is involved in the production of stomach prostaglandins, the maintenance of normal renal function, and the prevention of platelet aggregation. In contrast, COX-2 is normally absent from the body and is induced on-site in association with inflammation. Following induction, COX-2 produces large amounts of prostaglandins characteristically causing pain, fever, and peripheral vasodilation effecting local redness and edema formation. Selective inhibition of COX-2 would be advantageous to alleviate inflammation and associated pain without disturbing normal functions of the body.

TNF-alpha, along with other compounds such as, for example, histamines or interleukins, are inflammatory mediators. The use of one or more inhibitors or antagonists to TNF-alpha in a cosmetic, dermatological or pharmaceutical topical product would alleviate skin inflammation.

NGF is a well characterized member of the neurotrophin family that is secreted in the nervous system, but also in the skin. It is commonly known to cause inflammatory hyperalgesia and is elevated in various skin conditions that include atopic dermatitis. NGF is also known to activate mast cells and to regulate pro-inflammatory neuropeptides such as substance P. Inhibition of NGF would alleviate skin inflammation and the hyperalgesia associated with inflammation.

The present invention provides compositions having at least one plant extract in an amount effective to inhibit TNF-alpha protein, NGF protein and/or COX-2 enzyme.

The present invention in its broadest view encompasses the use in any topical cosmetic, dermatological, or pharmaceutical composition of any convenient plant extract or ingredient inhibitory to TNF-alpha protein, NGF protein and/or COX-2 enzyme to alleviate or treat any visible or subjective skin irritation. The phrase "skin irritation" includes, but is not limited to, the visible and/or subjective irritation of skin, including but not limited to erythema, psoriasis, edema, hyper-pigmentation, hypo-pigmentation, acne, warts, wheeling, blotchiness, uneven skin tone, scaling, flaking, itching, burning, stinging, tingling, numbing, wind irritation, temperature irritation, smoke irritation, and/or chemical irritation. The compositions of the present invention are also effective in treating subclinical irritation, i.e., where redness is not present, but where the skin is already compromised at a cellular level.

The one or more plant ingredients, preferably natural extracts, used in compositions of the present invention to inhibit COX-2 enzyme, NGF protein and/or TNF-alpha protein for treatment of skin irritation include one or more extracts, or natural ingredients. These extracts or natural ingredients are preferably any one or more of the following: *Ilex purpurea* Hassk, *Ligusticum chiangxiong, Osmunda japonica, Ligusticum licidum, Butea frondosa*, or *Mimusops elengi*. More preferably, these plant ingredients, preferably extracts, are one or more of the following: *Ilex purpurea* Hassk, *Ligusticum chiangxiong, Osmunda japonica, Butea frondosa,* or *Ligusticum lucidum*. The foregoing extracts are typically obtained by either hydrophilic or hydrophobic extraction of said plant or of its parts. Especially preferred is the combination of *Ligusticum chiangxiong* and *Butea frondosa.*

The amount of the plant extract in the present compositions is 0.0001 percentage by weight (wt%) to 99 wt%, and preferably 0.001 wt% to 20 wt%, based on the total weight of the composition. The amount of the plant extract is more preferably 0.01 wt% to 5 wt%, and still more preferably 0.1 wt% to 3 wt%, based on the total weight of the composition.

The present invention provides, as one embodiment, compositions for treatment of skin irritation having a cosmetically, dermatologically or pharmaceutically effective amount of at least one extract derived from the above plant sources sufficient to inhibit COX-2 enzyme, NGF protein and/or TNF-alpha protein. Preferably, such compositions also have a cosmetically, dermatologically or pharmaceutically acceptable vehicle. In addition, blends of such extracts may conveniently be employed. Embodiments of the present invention may conveniently be employed to treat various skin irritation symptoms or conditions (i.e., erythema, psoriasis, edema, hyper-pigmentation, hypo-pigmentation, acne, warts, wheeling, blotchiness, uneven skin tone, scaling, flaking, itching, burning, stinging, tingling, numbing, wind irritation, temperature irritation, smoke irritation, and chemical irritation).

In other embodiments of the present invention, compositions may have an active ingredient, or combination of active ingredients, in an amount that would normally produce skin irritation symptom or condition, but for the inclusion in such compositions of a cosmetically, dermatologically or pharmaceutically effective amount of one or more extracts derived from the above plant sources sufficient to inhibit COX-2 enzyme, NGF protein and/or TNF-alpha protein. Such extracts, or blends of extracts, and one or more active ingredients or combination of active ingredients in an amount that would normally produce skin irritation symptom or condition, are conveniently incorporated into a pharmaceutically or cosmetically acceptable vehicle in a form suitable for topical application.

Cosmetically, dermatologically or pharmaceutically acceptable vehicles that can be used in the present topical compositions include, but are not limited to, one or more aqueous systems, glycerins, C₁₋₄ alcohols, fatty alcohols, fatty ethers, fatty esters, polyols, glycols, vegetable oils, mineral oils, liposomes, laminar lipid materials, silicone oils, water or any combinations thereof.

In addition, the vehicle of the present compositions can be in the form of a homogeneous phase formulation or in the form of an emulsion. Such emulsions include, but not limited to, oil-in-water, water-in-oil, water-in-silicone, and multiple including triple, phase emulsions. These emulsions can cover a broad range of consistencies including thin lotions (which can also be suitable for spray or aerosol delivery), creamy lotions, light creams and heavy creams. Other suitable topical carriers include an anhydrous liquid solvent such as oil and alcohol; aqueous-based single-phase liquid solvent (e.g., hydro-alcoholic solvent system); anhydrous solid and semisolid (such as gel and stick); and aqueous based gel and mousse system. Examples of vehicles or vehicle systems that can be used in the present invention are described in the following four references:
"Sun Products Formulary", Cosmetics & Toiletries, vol. 105, pp. 122-139 (December 1990); "Sun Products Formulary", Cosmetics & Toiletries, vol. 102, pp. 117-136 (March 1997); U.S. Patent No. 4,960,764 to Figueroa et al., issued October 2, 1990; and U.S. Patent No. 4,254,105 to Fukuda et al., issued March 3, 1981.

The topical compositions of the present invention can be formulated in any suitable product form. Such product forms include, but are not limited to, aerosol spray, cream, emulsion, solid, liquid, dispersion, foam, gel, lotion, mousse, ointment, powder, patch, pomade, solution, pump spray, stick, and towelette. Product applications include all topical skin care product formulations, color cosmetics, personal care products (i.e., anti-perspirants, deodorants and the like), hair care products, and topical pharmaceutical products. Compositions of the present invention for use in or as cosmetic, dermatological, or pharmaceutical topical application products can conveniently be prepared by various methodologies well known in the art.

The present topical compositions may include one or more of the following: anesthetics, anti-allergenics, antifungals, antimicrobials, other anti-inflammatory agents, antioxidants, antiseptics, chelating agents, colorants, depigmenting agents, emollients, emulsifiers, exfollients, film formers, fragrances, humectants, insect repellents, lubricants, moisturizers, pharmaceutical agents, photostabilizing agents, preservatives, skin protectants, skin penetration enhancers, sunscreens, stabilizers, surfactants, thickeners, viscosity modifiers, vitamins, or any combinations thereof.

The present compositions provide for products, especially cosmetic products, which alleviate skin irritation. The present invention provides compositions having therapeutically specific and standardized supply of active ingredients alleviating skin irritation by inhibiting COX-2 enzyme, NGF protein and/or TNF-alpha protein. The present compositions can conveniently be formulated to deliver a consistent level of an active ingredient, or blend of ingredients, so that the desired effect of alleviation of skin irritation is achieved.

The present invention is further illustrated by the following Examples.

### Example 1

Various natural plant extracts of the present invention were evaluated for inhibition of COX-1 enzyme, NGF, and TNF-alpha in vitro studies as described below.

### Cyclooxygenase Inhibition Protocol

The efficacy of the plant extracts *Ligusticum chiangxiong Hort., Osmunda japonica Thunb., Ilex purpurea Hassk*, and *Butea frondosa Roxb*. for the inhibition of cyclooxygenase using a COX enzyme immunoassay (EIA) commercially available from Cayman Chemical under the trademark COX Inhibitor Screening Assay.

The COX reaction for each material tested was prepared with a background reaction (containing inactive COX enzyme) and an initial activity reaction (containing active enzyme with and without screening material). Following 37°C incubation for 10 minutes, arachidonic acid was added as a substrate for the COX enzyme. After 2 minutes incubation, 1M HCl was added to stop the reaction. Saturated stannous chloride solution was added (100 µL) to stabilize the prostaglandin in the reaction.

The EIA reaction was prepared by diluting the COX reactions. EIA buffer was added to some wells to generate Non-Specific Binding wells followed by addition of the Cox reactions. Prostaglandin (PG) screening acetylcholinesterase tracer was added to wells followed by addition of the PG antiserum to every well. The plate was incubated at room temperature for 18 hours and all wells. The Total Activity was spectrophotometrically measured at 405 nm. The results of the tests are provided in Table I below.

### TNF-alpha Inhibition Protocol

To test the efficacy of *Ilex pururea Hassk, Osmunda japonica Thunb., Butea frondosa Roxb*., and *Ligusticum chuangxiong* for the inhibition of TNF-alpha production, an enzyme linked immunoassay (ELISA) commercially available from R&D Systems was employed.

This assay employed the quantitative sandwich enzyme immunoassay technique in which a monoclonal antibody specific for TNF-alpha has been pre-coated onto a microtiter plate. Culture supernatants from cells exposed to active materials were pipetted into separate wells. TNF-alpha in the supernatant was bound to the plate via the immobilized antibody. After several washes to remove unbound antibody, an enzyme-linked polyclonal antibody specific for TNF-alpha was added to each well. Following a wash to remove unbound antibody-enzyme reagent, a substrate solution was added to the wells. Color develops in proportion to the amount of TNF-alpha bound in the initial step. The results of the tests are provided in Table I.

### NGF Inhibition Protocol

To test the efficacy of *Ilex purpurea Hassk, Osmunda japonica* was employed. The Nerve Growth Factor immunoassay is designed for the sensitive and specific detection of NGF. Flat-bottom microtiter plates are coated with Anti-NGF Polyclonal Antibody that binds soluble NGF. The captured NGF is bound by a second specific monoclonal antibody. After washing, the amount of specifically bound mAb is then detected using a species-specific antibody conjugated to horseradish peroxidase (HRP) as a tertiary reactant. The unbound conjugate is removed by washing. Following an incubation with a chromogenic substrate, the color change is measured. The amount of NGF in the test solutions is proportional to the color generated in the oxidation-reduction reaction. The results are provided in Table I.

**Table I**

| **Results of In Vitro Tests** | | | |
|---|---|---|---|
| Plant Extract | TNF-alpha Inhibition | COX-2 Inhibition | NGF Inhibition |
| *Ilex purpurea Hassk* | +++ | ++ | ++++ |
| *Osmunda japonica Thunb.* | +++ | (0) | ++ |
| *Butea frondosa Roxb*. | +++ | (0) | ++ |
| *Ligusticum chuangxiong Hort*. | +++ | (0) | |
| *Ligusticum lucidum* | +++ | | |
| *Mimusops elengi linn*. | +++ | | |

| | | | |
|---|---|---|---|
| Legend: +, ++, +++, ++++ Significant inhibition (degree of inhibition quantified by number of plus signs) (0) No change | | | |

These in vitro studies show that the compositions of the present invention containing an extract of: *Ilex purpurea Hassk, Ligusticum chiangxiong, Osmunda japonica,* or *Butea frondosa* provide benefits to the skin by inhibiting COX-2 enzyme, NGF protein and/or TNF-alpha protein activity, whereby the signs of subjective discomfort and/or irritation caused by cosmetic, pharmaceutical or dermatological products would be reduced, thus providing an improvement in skin appearance. These extracts or actives of the present invention are non-toxic at the amounts tested.

### Example 2

*Ligusticum chuangxiong* and *Butea frondosa* were evaluated in various biopsy studies in which the natural plant extract as set forth below was incorporated into a cosmetically suitable vehicle and applied to the volar forearm of a participant in the study, at a dose of 2 mg/cm². The forearm area to which the extract preparation was applied was then covered with a semi-occlusive patch. This procedure was repeated for 3 weeks, 5 days per week. At the end of the treatment the sites were anesthetized with lidocaine and 2 mm punch biopsies were taken from the treated and one untreated control site. Biopsies were fixed in formalin, embedded in paraffin, sectioned, and stained for relevant endpoints.

For *Ligusticum chianxiaong,* 7 of 8 panelists showed an increase in the number of epidermal and dermal nerve fibers (visualized with the marker PGP 9.5)

For *Butea frondosa,* 5 of 8 panelists showed increase in keratinocyte proliferation (visualized by the antibody to KI67) and 5 of 8 panelists showed increase in viable epidermal thickness.

### Example 3

Inflammation challenge studies for *Butea frondosa* were conducted as follows: The minimum erythemal dose (MED) for UV and SLS of a group of panelists was first determined. A composition containing *Butea frondosa* extract in a vehicle comprising 2 parts propylene glycol, 1 part ethyl alcohol, and 1 part water, was prepared. The compositions were applied to the skin of the back of test panelists and left to penetrate. The procedure was repeated for 5 consecutive days, after which an amount of UV (on one half of the back) and SLS (on the other half) (both sufficient to normally elicit erythema) was applied to the back. Visual grading of the erythema readings confirmed the anti-inflammatory activity benefit of the *Butea frondosa* extract in these pretreatment tests. A similar inflammation challenge study for *Ligusticum lucidum* was not conclusive.

Various alternatives and modifications of the present invention can be devised by those skilled in the art without departing from the invention. Accordingly, the present invention is intended to encompass all such alternatives, modifications and variances that fall within the scope of the following claims.

## Claims

1. A topical composition comprising:
at least one cosmetic, dermatological, or pharmaceutical active ingredient in an amount effective to provide its intended cosmetic, dermatological, or pharmaceutical response, said at least one active ingredient being known to elicit a skin irritation symptom or condition, wherein said active ingredient is selected from the group consisting of hydroxylated acids, α-hydroxy acids, β-hydrdxy acids, retinoids, anthralins, anthranoids, peroxides, minoxidil, lithium salts, antimetabolites, vitamin D, para-phenylenediamine, aminophenol, alcoholic perfuming solutions, antiperspirant agents, depilatory or hair permanent active agents, depigmentating agents, insecticide agents, and any combinations thereof;
at least one plant extract in an amount cosmetically, dermatologically, or pharmaceutically effective to alleviate such skin irritation symptom or condition elicited by said at least one active ingredient, wherein said at least one plant extract is selected from the group consisting of Ilex purpurea Hassk, Ligusticum chiangxiong, Osmunda japonica, Butea frondosa, and any combinations thereof; and
a cosmetically, dermatologically, or pharmaceutically acceptable vehicle.

2. The composition of claim 1, wherein said at least one plant extract Ligusticum chiangxiong.

3. The composition of claim 1, wherein said at least one plant extract is Osmunda japonica.

4. The composition of claim 1, wherein said at least one plant extract is Butea frondosa.

5. The composition of claim 1, wherein said at least one plant extract is Ilex purpurea Hassk.

6. The composition of claim 1, wherein said at least one plant extract is a blend of Butea frondosa and Ligusticum chiangxiong.

7. The composition of claim 1, wherein said at least one plant extract is present in an amount 0.001 wt% to 20 wt% based on the total weight of the composition.

8. The composition of claim 1, wherein said at least one plant extract is present in an amount 0.01 wt% to 5 wt% based on the total weight of the composition.

9. The composition of claim 1, wherein the composition is in a product form selected from the group consisting of an aerosol spray, cream, emulsion, solid, liquid, dispersion, foam, gel, lotion, mousse, ointment, powder, patch, pomade, solution, pump spray, stick, and towelette.

10. The composition of claim 1, wherein said skin irritation symptom or condition is at least one symptom or condition is selected from the group consisting of erythema, psoriasis, edema, hyper-pigmentation, hypo-pigmentation, acne, warts, wheeling, blotchiness, uneven skin tone, scaling, flaking, itching, burning, stinging, tingling, numbing, wind irritation, temperature irritation, smoke irritation, chemical irritation, and any combinations thereof.

11. The composition of claim 1, wherein said active ingredient is selected from the group consisting of hydroxylated acid, α-hydroxy acid, β-hydroxy acid, retinoid, and any combinations thereof.

12. The composition of claim 11, wherein said active ingredient is at least one retinoid selected from the group consisting of retinoid acid and retinol.

13. The composition of claim 12, wherein said at least one retinoid is retinol.

14. The composition of claim 11, wherein said active ingredient is at least one α-hydroxy acid selected from the group consisting of lactic acid, glycolic acid, citric acid, malic acid, tartaric acid, mandelic acid, atrolactic acid, gluconic acid, methyl lactic acid, phenyl lactic acid, glyceric acid, benzilic acid, 2-hydroxyheptanoic acid, 2-hydroxyoctanoic acid, and any combinations thereof.

15. The composition of claim 11, wherein said active ingredient is at least one β-hydroxy acid selected from the group consisting of salicylic acid, and 5-n-octanoylsalicylic acid.

16. A non-therapeutic method for improving the aesthetic appearance of skin comprising topically applying to the skin, a cosmetically effective amount of a topical composition comprising:
at least one cosmetic active ingredient in an amount effective to provide its intended cosmetic response, wherein said active ingredient is selected from the group consisting of hydroxylated acids and α-hydroxy acids, β-hydroxy acids, retinoids, peroxides, vitamin D and, para-phenylendiamine, aminophenol, alcoholic perfuming solutions, antiperspirant agents, depilatory or hair permanent active agents, depigmentating agents, insecticide agents, and any combinations thereof;
at least one plant extract in a cosmetically effective amount, wherein said at least one plant extract is selected from the group consisting of Ilex purpurea Hassk, Ligusticum chiangxiong, Osmunda japonica, Butea frondosa, and any combinations thereof; and
a cosmetically acceptable vehicle;
wherein at least one symptom of the following group of symptons is treated cosmetically;
a) reducing or preventing loss of collagen;
b) improving skin firmness/plumpness;
c) improving skin texture;
d) decreasing/preventing lines and wrinkles;
e) improving skin tone;
f) enhancing skin thickness;
g) decreasing pore size;
h) reducing skin discoloration;
i) reducing acne;
j) reducing skin sensitivity;

17. The method of claim 16, wherein said at least one plant extract is of Butea frondosa.

18. The method of claim 16, wherein said at least one plant extract is Ilex purpurea Hassk.

19. The method of claim 16, wherein said at least one plant extract is Ligusticum chiangxiong.

20. The method of claim 16, wherein said at least one plant extract is Osmunda japonica.

21. The method of claim 16, wherein said at least one plant extract is present in an amount 0.001 wt% to 20 wt% based on the total weight of the composition.

22. The method of claim 16, wherein said at least one plant extract is present in an amount 0.01 wt% to 5 wt% based on the total weight of the composition.

23. The composition of claim 16, wherein said active ingredient is selected from the group consisting of hydroxylated acid and, α-hydroxy acid, β-hydroxy acid, retinoid, and any combinations thereof.

24. The method of claim 23, wherein said active ingredient is at least one retinoid selected from the group consisting of retinoid acid and retinol.

25. The method of claim 24, wherein said at least one retinoid is retinol.

26. The method of claim 16, wherein the α-hydroxy acid is selected from the group consisting of lactic acid, glycolic acid, citric acid, malic acid, tartaric acid, mandelic acid, atrolactic acid, gluconic acid, methyl lactic acid, phenyl lactic acid, glyceric acid, benzilic acid, 2-hydroxyheptanoic acid, 2-hydroxyoctanoic acid, and any combinations thereof.

27. The method of claim 23, wherein the β-hydroxy acid is selected from the group consisting of salicylic acid and 5-n-octanoylsalicylic acid.

## Patentansprüche

1. Topische Zusammensetzung, Folgendes umfassend:
mindestens einen kosmetischen, dermatologischen oder pharmazeutischen Wirkstoff in einer Menge, die ausreicht, die beabsichtigte kosmetische, dermatologische oder pharmazeutische Reaktion auszulösen, wobei von dem mindestens einen Wirkstoff bekannt ist, dass er ein Hautreizungssymptom oder einen Hautreizungszustand auslöst, wobei der Wirkstoff ausgewählt ist aus der Gruppe bestehend aus hydroxylierten Säuren, α-Hydroxycarbonsäuren, β-Hydroxycarbonsäuren, Retinoiden, Anthralinen, Anthranoiden, Peroxiden, Minoxidil, Lithiumsalzen, Antimetaboliten, Vitamin D, Para-Phenylendiamin, Aminophenol, alkoholischen parfümierenden Lösungen, transpirationshemmenden Mitteln, haarentfernenden oder haarpermanenten Wirkstoffen, depigmentierenden Mitteln, Insektiziden und beliebigen Kombinationen hiervon;
mindestens ein Pflanzenextrakt in einer kosmetisch, dermatologisch oder pharmazeutisch wirksamen Menge, die geeignet ist, derartige Hautreizungssymptome oder Zustände, die von dem mindestens einen Wirkstoff ausgelöst wurden, zu mildern, wobei das mindestens eine Pflanzenextrakt aus der Gruppe bestehend aus Ilex purpurea Hassk, Ligusticum chiangxiong, Osmunda japonica, Butea frondosa und aus beliebigen Kombination hiervon ausgewählt ist; und eine kosmetisch, dermatologisch oder pharmazeutisch akzeptable Trägersubstanz.

2. Zusammensetzung gemäß Anspruch 1, wobei das mindestens eine Pflanzenextrakt Ligusticum chiangxiong ist.

3. Zusammensetzung gemäß Anspruch 1, wobei das mindestens eine Pflanzenextrakt Osmunda japonica ist.

4. Zusammensetzung gemäß Anspruch 1, wobei das mindestens eine Pflanzenextrakt Butea frondosa ist.

5. Zusammensetzung gemäß Anspruch 1, wobei das mindestens eine Pflanzenextrakt Ilex purpurea Hassk ist.

6. Zusammensetzung gemäß Anspruch 1, wobei das mindestens eine Pflanzenextrakt eine Mischung aus Butea frondosa und Ligusticum chiangxiong ist.

7. Zusammensetzung gemäß Anspruch 1, wobei das mindestens eine Pflanzenextrakt in einer Menge von 0,001 Gew.-% bis 20 Gew.-% auf Basis des Gesamtgewichts der Zusammensetzung vorhanden ist.

8. Zusammensetzung gemäß Anspruch 1, wobei das mindestens eine Pflanzenextrakt in einer Menge von 0,01 Gew.-% bis 5 Gew.-% auf Basis des Gesamtgewichts der Zusammensetzung vorhanden ist.

9. Zusammensetzung gemäß Anspruch 1, wobei die Zusammensetzung in einer Produktform vorliegt, die aus der Gruppe bestehend aus einem Aerosol-Spray, einer Creme, einer Emulsion, einem Feststoff, einer Flüssigkeit, einer Dispersion, einem Schaum, einem Gel, einer Lotion, einem Mousse, einer Salbe, einem Pulver, einem Pflaster, einer Pomade, einer Lösung, einem Pumpenspray, einem Stick und einem Erfrischungstuch ausgewählt ist.

10. Zusammensetzung gemäß Anspruch 1, wobei das Hautreizungssymptom oder der Zustand mindestens ein Symptom oder Zustand ist, der aus folgender Gruppe ausgewählt ist: Erythem, Psoriasis, Ödem, Hyperpigmentierung, Hypopigmentierung, Akne, Warze, Quaddel, Fleckenbildung, ungleichmäßiger Teint, Schuppen, Hautabblättern, Jucken, Brennen, Stechen, Prickeln, Gefühllosigkeit, Windreizung, Temperaturreizung, Rauchreizung, chemische Reizung und beliebige Kombinationen hiervon.

11. Zusammensetzung gemäß Anspruch 1, wobei der Wirkstoff ausgewählt ist aus der Gruppe bestehend aus hydroxylierter Säure, α-Hydroxycarbonsäure, β-Hdyroxycarbonsäure, Retinoid und einer beliebigen Kombination hiervon.

12. Zusammensetzung gemäß Anspruch 11, wobei der Wirkstoff mindestens ein Retinoid ist, das aus der Gruppe bestehend aus Retinoidsäure und Retinol ausgewählt ist.

13. Zusammensetzung gemäß Anspruch 12, wobei das mindestens eine Retinoid Retinol ist.

14. Zusammensetzung gemäß Anspruch 11, wobei der Wirkstoff mindestens eine α-Hydroxycarbonsäure ist, die aus der Gruppe bestehend aus Milchsäure, Glykolsäure, Citronensäure, Apfelsäure, Weinsäure, Mandelsäure, Atrolactinsäure, Gluconsäure, Methylmilchsäure, Phenylmilchsäure, Glycersäure, Benzilsäure, 2-Hydroxyheptansäure, 2-Hydroxyoctansäure und beliebigen Kombination hieraus ausgewählt ist.

15. Zusammensetzung gemäß Anspruch 11, wobei der Wirkstoff mindestens eine β-Hydroxycarbonsäure ist, die aus der Gruppe bestehend aus Salicylsäure und 5-n-Octanoylsalicylsäure ausgewählt ist.

16. Nicht-therapeutisches Verfahren zur Verbesserung der ästhetischen Erscheinung der Haut, umfassend das topische Auftragen einer kosmetisch wirksamen Menge einer topischen Zusammensetzung auf die Haut, wobei die Zusammensetzung Folgendes umfasst:
mindestens einen kosmetischen Wirkstoff in einer Menge, die ausreicht, die beabsichtigte kosmetische Reaktion hervorzurufen, wobei der Wirkstoff ausgewählt ist aus der Gruppe bestehend aus hydroxylierten Säuren und α-Hydroxycarbonsäuren, β-Hydroxycarbonsäuren, Retinoiden, Peroxiden, Vitamin D, Para-Phenylendiamin, Aminophenol, alkoholischen parfümierenden Lösungen, transpirationshemmenden Mitteln, haarentfernenden oder haarpermanenten Wirkstoffen, depigmentierenden Mitteln, Insektiziden und beliebigen Kombinationen hiervon;
mindestens ein Pflanzenextrakt in einer kosmetisch wirksamen Menge, wobei das mindestens eine Pflanzenextrakt aus der Gruppe bestehend aus Ilex purpurea Hassk, Ligusticum chiangxiong, Osmunda japonica, Butea frondosa und aus beliebigen Kombination hiervon ausgewählt ist; und
eine kosmetisch akzeptable Trägersubstanz;
wobei mindestens eines aus der folgenden Gruppe von Symptomen kosmetisch behandelt wird:
a) Reduzierung oder Verhinderung von Kollagenverlust;
b) Verbesserung der Hautfestigkeit/Plumpheit;
c) Verbesserung der Hauttextur;
d) Verringerung/Vermeidung von Falten und Runzeln;
e) Verbesserung des Haut-Teints;
f) Erhöhung der Hautdicke;
g) Verringerung der Porengröße;
h) Verringerung der Hautverfärbung;
i) Verringerung von Akne;
j) Verringerung der Hautempfindlichkeit.

17. Verfahren gemäß Anspruch 16, worin das mindestens eine Pflanzenextrakt von Butea frondosa stammt.

18. Verfahren gemäß Anspruch 16, worin das mindestens eine Pflanzenextrakt Ilex purpurea Hassk ist.

19. Verfahren gemäß Anspruch 16, worin das mindestens eine Pflanzenextrakt Ligusticum chiangxiong ist.

20. Verfahren gemäß Anspruch 16, worin das mindestens eine Pflanzenextrakt Osmunda japonica ist.

21. Verfahren gemäß Anspruch 16, worin das mindestens eine Pflanzenextrakt in einer Menge von 0,001 Gew.-% bis 20 Gew.-% auf Basis des Gesamtgewichts der Zusammensetzung vorhanden ist.

22. Verfahren gemäß Anspruch 16, worin das mindestens eine Pflanzenextrakt in einer Menge von 0,01 Gew.-% bis 5 Gew.-% auf Basis des Gesamtgewichts der Zusammensetzung vorhanden ist.

23. Zusammensetzung gemäß Anspruch 16, wobei der Wirkstoff aus der Gruppe bestehend aus hydroxylierter Säure und α-Hydroxycarbonsäure, β-Hydroxycarbonsäure, Retinoid und beliebigen Kombination hiervon ausgewählt ist.

24. Verfahren gemäß Anspruch 23, wobei der Wirkstoff mindestens ein Retinoid ist, das aus der Gruppe bestehend aus Retinoidsäure und Retinol ausgewählt ist.

25. Verfahren gemäß Anspruch 24, wobei das mindestens eine Retinoid Retinol ist.

26. Verfahren gemäß Anspruch 16, wobei die α-Hydroxycarbonsäure aus der Gruppe bestehend aus Milchsäure, Glykolsäure, Citronensäure, Apfelsäure, Weinsäure, Mandelsäure, Atrolactinsäure, Gluconsäure, Methylmilchsäure, Phenylmilchsäure, Glycersäure, Benzilsäure, 2-Hydroxyheptansäure, 2-Hydroxyoctansäure und beliebigen Kombinationen hieraus ausgewählt ist.

27. Verfahren gemäß Anspruch 23, wobei die β-Hydroxycarbonsäure aus der Gruppe bestehend aus Salicylsäure und 5-n-Octanoylsalicylsäure ausgewählt ist.

## Revendications

1. Composition pour application locale comprenant :
au moins un ingrédient actif ayant une action cosmétique, dermatologique ou pharmaceutique dans une quantité efficace pour produire son action cosmétique, dermatologique ou pharmaceutique, lequel au moins un ingrédient actif est connu pour provoquer un symptôme ou un état d'irritation cutanée, ledit ingrédient actif étant choisi parmi les acides hydroxylés, les acides α-hydroxylés, les acides β-hydroxylés, les rétinoïdes, le anthralines, les anthranoïdes, les peroxydes, le minoxidil, les sels de lithium, les antimétabolites, la vitamine D, la paraphénylènediamine, l'aminophénol, les solutions parfumées alcooliques, les agents antitranspirants, les agents actifs dépilatoires ou pour permanentes capillaires, les agents dépigmentants, les insecticides et toute combinaison de ceux-ci ;
au moins un extrait végétal dans une quantité efficace du point de vue cosmétique, dermatologique ou pharmaceutique pour atténuer ce symptôme ou cet état d'irritation cutanée provoqué par ledit au moins un ingrédient actif, ledit au moins un extrait végétal étant sélectionné parmi *Ilex purpurea* Hassk, *Ligusticum chiangxiong, Osmunda japonica, Butea frondosa*, et toute combinaison de ceux-ci ; et
un véhicule acceptable du point de vue cosmétique, dermatologique ou pharmaceutique.

2. Composition selon la revendication 1, dans laquelle ledit au moins un extrait végétal est *Ligusticum chiangxiong.*

3. Composition selon la revendication 1, dans laquelle ledit au moins un extrait végétal est *Osmunda japonica.*

4. Composition selon la revendication 1, dans laquelle ledit au moins un extrait végétal est *Butea frondosa.*

5. Composition selon la revendication 1, dans laquelle ledit au moins un extrait végétal est *Ilex purpurea* Hassk.

6. Composition selon la revendication 1, dans laquelle ledit au moins un extrait végétal est un mélange de *Butea frondosa* et *Ligusticum chiangxiong.*

7. Composition selon la revendication 1, dans laquelle ledit au moins un extrait végétal est présent à raison de 0,001 % en poids à 20 % en poids sur la base du poids total de la composition.

8. Composition selon la revendication 1, dans laquelle ledit au moins un extrait végétal est présent à raison de 0,01 % en poids à 5 % en poids sur la base du poids total de la composition.

9. Composition selon la revendication 1, dans laquelle la composition se présente sous la forme d'un produit sélectionné parmi un aérosol, une crème, une émulsion, un solide, un liquide, une dispersion, une mousse, un gel, une lotion, une mousse, un onguent, une poudre, un patch, une pommade, un flacon pulvérisateur, un stick et une lingette.

10. Composition selon la revendication 1, dans laquelle ledit symptôme ou état d'irritation cutanée est au moins un symptôme ou état choisi parmi l'érythème, le psoriasis, l'oedème, l'hyperpigmentation, l'hypopigmentation, l'acné, les verrues, les papules, les taches cutanées, l'irrégularité du teint, les desquamations, les pellicules, les prurits, les brûlures, les piqûres, les picotements, les insensibilités, l'irritation par le vent, l'irritation par la température, l'irritation par la fumée, l'irritation chimique et toute combinaison de ceux-ci.

11. Composition selon la revendication 1, dans laquelle ledit ingrédient actif est choisi parmi un acide hydroxylé, un acide α-hydroxylé, un acide β-hydroxylé, un rétinoïde et toutes combinaisons de ceux-ci.

12. Composition selon la revendication 11, dans laquelle ledit ingrédient actif est au moins un rétinoïde choisi parmi l'acide rétinoïque et le rétinol.

13. Composition selon la revendication 12, dans laquelle ledit au moins un rétinoïde est du rétinol.

14. Composition selon la revendication 11, dans laquelle ledit ingrédient actif est au moins un acide α-hydroxylé choisi parmi l'acide lactique, l'acide glycolique, l'acide citrique, l'acide malique, l'acide tartrique, l'acide mandélique, l'acide atrolactique, l'acide gluconique, l'acide méthyl-lactique, l'acide phényl-lactique, l'acide glycérique, l'acide benzylique, l'acide 2-hydroxyheptanoïque, l'acide 2-hydroxyoctanoïque et toute combinaison de ceux-ci.

15. Composition selon la revendication 11, dans laquelle ledit ingrédient actif est au moins un acide β-hydroxylé choisi parmi l'acide salicylique et l'acide 5-n-octanoylsalicylique.

16. Méthode non thérapeutique pour améliorer l'aspect esthétique de la peau, comprenant l'application locale sur la peau d'une quantité efficace du point de vue cosmétique d'une composition pour application locale comprenant :
au moins un ingrédient actif ayant une action cosmétique dans une quantité efficace pour produire son action cosmétique prévue, lequel ingrédient actif est choisi parmi les acides hydroxylés, les acides α-hydroxylés, les acides β-hydroxylés, les rétinoïdes, les peroxydes, la vitamine D, la paraphénylènediamine, l'aminophénol, les solutions parfumées alcooliques, les agents antitranspirants, les agents actifs dépilatoires ou pour permanentes capillaires, les agents dépigmentants, les insecticides et toute combinaison de ceux-ci ;
au moins un extrait végétal dans une quantité efficace du point de vue cosmétique, lequel au moins un extrait végétal est sélectionné parmi *Ilex purpurea* Hassk, *Ligusticum chiangxiong, Osmunda japonica, Butea frondosa*, et toute combinaison de ceux-ci ; et
un véhicule acceptable du point de vue cosmétique ;
dans laquelle au moins un symptôme dans le groupe suivant de symptômes fait l'objet d'un traitement cosmétique :
a) réduction ou prévention de la perte de collagène ;
b) amélioration de la fermeté de la peau ;
c) amélioration de la texture de la peau ;
d) réduction/prévention des ridules et des rides ;
e) amélioration du teint ;
f) augmentation de l'épaisseur de la peau ;
g) réduction de la taille des pores ;
h) réduction des décolorations cutanées ;
i) réduction de l'acné ;
j) réduction de la sensibilité cutanée.

17. Méthode selon la revendication 16, dans laquelle ledit au moins un extrait végétal est *Butea frondosa.*

18. Méthode selon la revendication 16, dans laquelle ledit au moins un extrait végétal est *Ilex purpurea* Hassk.

19. Méthode selon la revendication 16, dans laquelle ledit au moins un extrait végétal est *Ligusticum chiangxiong.*

20. Méthode selon la revendication 16, dans laquelle ledit au moins un extrait végétal est *Osmunda japonica.*

21. Méthode selon la revendication 16, dans laquelle ledit au moins un extrait végétal est présent à raison de 0,001 % en poids à 20 % en poids sur la base du poids total de la Méthode.

22. Méthode selon la revendication 16, dans laquelle ledit au moins un extrait végétal est présent à raison de 0,01 % en poids à 5 % en poids sur la base du poids total de la Méthode.

23. Méthode selon la revendication 16, dans laquelle ledit ingrédient actif est choisi parmi un acide hydroxylé et un acide α-hydroxylé, un acide β-hydroxylé, un rétinoïde et toutes combinaisons de ceux-ci.

24. Méthode selon la revendication 23, dans laquelle ledit ingrédient actif est au moins un rétinoïde choisi parmi l'acide rétinoïque et le rétinol.

25. Méthode selon la revendication 24, dans laquelle ledit au moins un rétinoïde est du rétinol.

26. Méthode selon la revendication 16, dans laquelle l'acide α-hydroxylé est choisi parmi l'acide lactique, l'acide glycolique, l'acide citrique, l'acide malique, l'acide tartrique, l'acide mandélique, l'acide atrolactique, l'acide gluconique, l'acide méthyl-lactique, l'acide phényl-lactique, l'acide glycérique, l'acide benzylique, l'acide 2-hydroxyheptanoïque, l'acide 2-hydroxyoctanoïque et toute combinaison de ceux-ci.

27. Méthode selon la revendication 23, dans laquelle l'acide β-hydroxylé est choisi parmi l'acide salicylique et l'acide 5-n-octanoylsalicylique.
